(19) Europäisches Patentamt
European Patent Office
Office européen des brevets

(11) EP 0 863 863 B1

(12) **FASCICULE DE BREVET EUROPEEN**

(45) Date de publication et mention de la délivrance du brevet:
**03.11.1999 Bulletin 1999/44**

(21) Numéro de dépôt: **95929145.1**

(22) Date de dépôt: **29.08.1995**

(51) Int Cl.6: **C07C 21/18**, C07C 17/26

(86) Numéro de dépôt international:
**PCT/FR95/01131**

(87) Numéro de publication internationale:
**WO 97/08118 (06.03.1997 Gazette 1997/11)**

(54) **NOUVEAUX COMPOSES ORGANIQUES FLUORES, APPLICATIONS EN PARTICULIER OPHTALMOLOGIQUES ET PROCEDE DE FABRICATION**

ORGANISCHE FLUORVERBINDUNGEN, IHRE VERWENDUNGEN, INSBESONDERE OPHTHALMISCHE UND VERFAHREN ZUR HERSTELLUNG

NOVEL FLUORINATED ORGANIC COMPOUNDS, OPHTHALMOLOGICAL APPLICATIONS THEREOF AND METHOD FOR MAKING SAME

(84) Etats contractants désignés:
**AT BE CH DE DK ES FR GB GR IE IT LI LU MC NL PT SE**

(43) Date de publication de la demande:
**16.09.1998 Bulletin 1998/38**

(73) Titulaire: **Chauvin Opsia**
**31320 Castanet-Tolosan (FR)**

(72) Inventeurs:
- **LATTES, Isabelle**
  **F-31520 Ramonville-Saint-Agne (FR)**
- **FEURER, Bernard**
  **F-31450 Montlaur (FR)**
- **GUIDETTI, Brigitte**
  **F-31130 Flourens (FR)**
- **PAYROU, Viviane**
  **F-31400 Toulouse (FR)**

(74) Mandataire:
**Cabinet BARRE LAFORGUE & associés**
**95, rue des Amidonniers**
**31000 Toulouse (FR)**

(56) Documents cités:
**EP-A- 0 545 174** **FR-A- 2 716 678**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 863 863 B1

Printed by Jouve, 75001 PARIS (FR)

## Description

L'invention concerne de nouveaux composés organiques fluorés possédant une chaîne fluorée et une chaîne hydrogénée ; elle vise des applications de ces nouveaux composés, en particulier applications ophtalmologiques ou comme milieu de conservation cellulaire et s'étend au procédé de fabrication de ces composés.

On sait que les composés fluorés présentent des propriétés spécifiques telles que bonne stabilité, biocompatibilité, capacité à solubiliser les gaz et notamment l'oxygène... qui conditionnent leur utilisation dans de nombreuses applications.

Parmi ces composés fluorés, on a préparé récemment des composés particuliers ayant une chaîne hydrogénée à double liaison : $R_F- CH_2- CH = CH - R_H$ où $R_F$ est une chaîne carbonée fluorée et $R_H$ une chaîne linéaire hydrogénée saturée. On pourra se reporter aux publications suivantes visant ce type de composés linéaires et leurs applications comme substituts du sang ou produits de remplacement de l'humeur vitrée de l'oeil : C. Cecutti et al., POTENTIAL BLOOD SUBSTITUTES SYNTHESIS AND MICROEMULSIFICATION OF MIXED HYDROGENATED AND FLUORINATED OILS, J. DISPERSION SCIENCE AND TECHNOLOGY, 7(3), 307-318 (1986) ; C. Cecutti et al., NEW FORMULATION OF BLOOD SUBSTITUTES : OPTIMIZATION OF NOVEL FLUORINATED MICROEMULSIONS, Eur. J. Med. Chem. 24 (1989) 485-492 ; C. Cecutti et al., A NEW FORMULATION FOR ELOOD SUBSTITUTES, J. DISPERSION SCIENCE AND TECHNOLOGY, 11(2), 115-123 (1990) ; Anne NOVELLI, Thèse MICROEMULSIFICATION ET BIOCOMPATIBILITE D'UN RADIOPROTECTEUR HYDROPHILE ET D'UN TRANSPORTEUR D'OXYGENE HYDROPHOBE, I.N.P.T., soutenance du 18 mai 1990.

Cette famille de composés fluorés linéaires présente des propriétés spécifiques de stabilité, de densité et de solubilisation de l'oxygène, qui, dans diverses applications, en rendent l'utilisation avantageuse par rapport aux autres composés fluorés. Ces propriétés proviennent, d'une part, de la double liaison centrale, d'autre part, de la chaîne hydrogénée disposée à l'opposé de la chaîne fluorée. La double liaison réduit la volatilité du composé à nombre de carbones égal et augmente sa capacité à solubiliser l'oxygène. La présence de la chaîne hydrogénée conduit à une densité fonction de la longueur de cette chaîne, plus faible que dans le cas de composés fluorés non hydrogénés possédant la même chaîne fluorée. Le choix conjugué de la chaîne fluorée $R_F$ et de la chaîne hydrogénée $R_H$ permet ainsi, dans une certaine mesure, d'obtenir des composés liquides de densité désirée, ayant un nombre de fluors désiré, c'est-à-dire une capacité donnée à solubiliser l'oxygène.

Ces composés fluorés linéaires sont actuellement préparés par un procédé mettant en oeuvre la réaction de Wittig. Toutefois, le rendement de cette synthèse est faible ; par exemple, les rendements moyens de synthèse pour des chaînes fluorées à huit carbones sont de l'ordre de 50 % (rendement global). Or, les produits de départ utilisés dans la réaction de Wittig sont des produits onéreux, de sorte que le coût des composés fluorés linéaires obtenus est très élevé, ce qui en limite considérablement les possibilités d'utilisation. A la connaissance des inventeurs, ces composés linéaires ont été uniquement synthétisés en laboratoire dans un but de recherche et ne sont pas utilisés. De plus, ces composés linéaires présentent de bonnes propriétés de solubilisation de l'oxygène à condition que leur chaîne fluorée possède un nombre de carbones relativement élevé, ce qui correspond à des composés de forte densité. Pour de nombreuses applications, le compromis densité/solubilisation de l'oxygène ne serait donc pas facile à trouver avec ce type de composés.

La présente invention se propose de fournir de nouveaux composés organiques fluorés qui présentent des propriétés au moins aussi intéressantes que les composés fluorés linéaires sus-évoqués, mais qui bénéficient de rendements de synthèse plus élevés, permettant de réduire leurs coûts de fabrication dans des proportions notables et élargissant donc leur possibilité d'utilisation.

Un autre objectif de l'invention est de fournir des composés fluorés ayant des propriétés de solubilisation de l'oxygène accrues par rapport aux composés linéaires sus-évoqués possédant la même chaîne fluorée.

Un autre objectif de l'invention est de faciliter dans chaque application l'ajustement des propriétés physico-chimiques du composé en fonction de l'exigence de l'application.

A cet effet, les nouveaux composés conformes à l'invention possèdent la structure moléculaire suivante :

$$(1) \qquad R_F - CH_2 - CH = \underset{\substack{|\\ R_{1H}}}{C} - R_{2H}$$

où $R_F$ est une chaîne carbonée fluorée, $R_{1H}$ est un hydrogène ou une chaîne carbonée hydrogénée saturée, $R_{2H}$ est un hydrogène ou une chaîne carbonée hydrogénée saturée, $R_{1H}$ et $R_{2H}$ ne sont pas simultanément des hydrogènes, le groupement

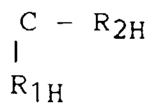

comporte une chaîne principale et au moins une ramification linéaire ou cyclique.

Ainsi, les composés organiques conformes à la présente invention sont caractérisés par la combinaison des éléments suivants :

- la présence d'une double liaison non réactive centrale dans la molécule,
- la présence de part et d'autre de la double liaison, d'une part d'une chaîne fluorée, d'autre part d'une chaîne hydrogénée,
- le caractère ramifié de la chaîne hydrogénée.

Ces composés sont liquides à température ambiante, parfaitement biocompatibles, de densité, capacité de solubiliser l'oxygène et indice de réfraction variables en fonction de leurs chaîne fluorée et chaîne hydrogénée ramifiée. On verra que leur capacité de solubiliser l'oxygène est notablement supérieure à celle des composés linéaires comparables (même chaîne fluorée, nombre total de carbones comparable) et qu'ils peuvent être fabriqués à moindre coût.

Dans une sous-famille, la partie $R_{1H}$ peut être constituée par un hydrogène, la structure moléculaire du composé étant la suivante :

(2) 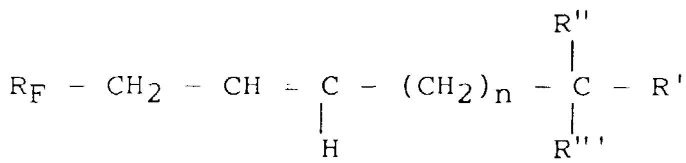

où les groupements R', R" et R"', identiques ou non, ont chacun la forme suivante : $(CH_2)_m$ - $C_xH_y$ avec :

$$n \geq 0$$

$$m \geq 0$$

$$y = 2x+1 \text{ avec } x > 0$$

au moins deux des groupements R', R" et R"' n'étant pas des hydrogènes,
le nombre total de carbones des groupements R', R" et R"' étant inférieur à 25-n.

Le groupement $C_xH_y$ peut notamment être constitué par un ou des radicaux méthyle, éthyle ou propyle, de façon à conduire à des composés qui, comme on le verra ci-après, ont des propriétés très avantageuses dans les applications ophtalmologiques, en particulier les trois composés de structure suivante :

(3) 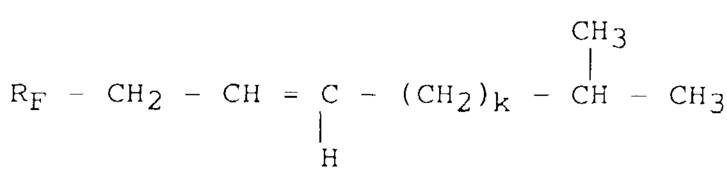

où k = 1, 2 ou 3.

Le composé suivant de la sous-famille sus-évoquée présente un indice de réfraction élevé, de l'ordre de 1,37 , qui le rend particulièrement intéressant dans les applications ophtalmologiques en raison de la différence de cet indice par rapport à celui du sérum physiologique (1,33), ce qui facilite son utilisation (injection et retrait mieux maîtrisés grâce à une parfaite visualisation du produit) :

$$(4) \qquad R_F - CH_2 - CH = CH - \underset{\underset{CH_3}{|}}{CH} - (CH_2)_8CH_3$$

Dans une autre sous-famille, les parties $R_{1H}$ et $R_{2H}$ sont, toutes deux, autres que des hydrogènes, les composés présentant la structure moléculaire suivante :

$$(5) \qquad R_F - CH_2 - CH = \underset{\underset{C_{x'}H_{y'}}{|}}{C} - C_xH_y$$

ou

$$y = 2x+1 \text{ avec } x \geq 1$$

$$y' = 2x'+1 \text{ avec } x' \geq 1$$

Dans cette sous-famille également, les groupements $C_xH_y$ et $C_{x'}H_{y'}$ peuvent être des radicaux méthyle, éthyle ou propyle.

La chaîne fluorée $R_F$ peut être une chaîne perfluorée ou non, comportant éventuellement un hétéroatome. La présence d'une chaîne perfluorée de formule $C_pF_{2p+1}$ avec p compris entre 2 et 12 est préférée dans diverses applications en raison de sa meilleure stabilité. Les composés ramifiés possédant cette chaîne ont une densité comprise entre 1,1 et 2,2, fonction croissante de p (et, dans une moindre mesure, fonction décroissante du nombre de carbones de la chaîne ramifiée hydrogénée). L'indice de réfraction de ces composés ramifiés à chaîne perfluorée est compris entre 1,2 et 1,7, fonction de p et du nombre de carbones des parties $R_{1H}$ et $R_{2H}$. La solubilité de ces composés dans l'huile de silicone est supérieure à 10 % en poids.

Les nouveaux composés fluorés ramifiés conformes à l'invention peuvent être préparés en utilisant une réaction de Wittig : de façon inattendue, on a pu constater que les rendements globaux de synthèse sont plus élevés que dans le cas des composés linéaires connus ayant la même chaîne fluorée. Ce résultat est difficilement explicable actuellement, mais on peut supposer qu'il est dû à une volatilité plus faible des composés ramifiés. Par exemple, dans le cas où la chaîne $R_F$ est constituée par la chaîne perfluorée à huit carbones $C_8F_{17}$, le rendement global de synthèse est d'environ 70 % (soit une augmentation relative de 40 % par rapport au rendement de synthèse des composés linéaires comparables) ; en outre, on constate une meilleure reproductibilité des résultats, la synthèse des composés linéaires étant très dépendante des conditions de mise en oeuvre de la réaction. De plus, on a pu mettre en évidence que, à nombre de carbones équivalent, la capacité à solubiliser l'oxygène est significativement plus élevée pour les composés ramifiés de l'invention (par rapport aux composés linéaires de même nombre de carbones). Ceci peut être expliqué par le fait que, dans le cas des composés conformes à l'invention, cette capacité à solubiliser l'oxygène provient, non seulement de la chaîne fluorée mais également, par un effet d'encombrement stérique, des parties ramifiées de la molécule.

Dans son mode de mise en oeuvre général, le procédé de fabrication des composés fluorés ramifiés conformes à l'invention comprend les opérations suivantes :

. on fait réagir à chaud une phosphine $Z_3P$ sur un halogénure de formule $XCH_2CH_2R_F$, de façon à obtenir un sel de phosphonium $Z_3P^+X^-CH_2CH_2R_F$,

. et on fait réagir à chaud dans un solvant ledit sel de phosphonium sur un aldéhyde ramifié ou une cétone

$$O = \underset{\underset{R_{1H}}{|}}{C} - R_{2H}$$

en présence d'une base faible.

Pour préparer un composé de la sous-famille (2), on fait réagir à chaud le sel de phosphonium sur un aldéhyde

ramifié de structure suivante :

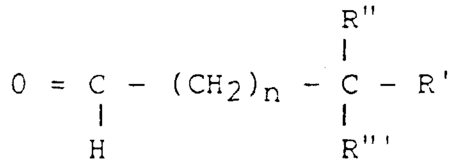

Dans le cas des trois composés (3) déjà évccués, on peut choisir, comme phosphine, la triphénylphosphine $(C_6H_5)_3$ P et, comme halogénure, un iodure de fluoroalkyle $I(CH_2)_2$ $R_F$. Le sel de phosphonium obtenu est ensuite amené à réagir avec l'aldéhyde ramifié suivant :

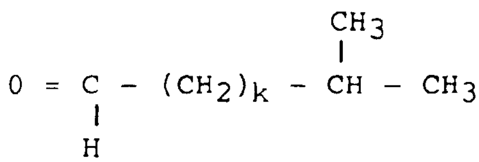

avec k = 1, 2 ou 3. On utilise de préférence comme solvant le dioxane en présence de carbonate de potassium et d'eau jouant le rôle de catalyseur. La température de réaction est alors ajustée à la température de reflux du dioxane. Le carbonate de potassium peut ensuite être éliminé par filtration, et l'oxyde de triphénylphosphine formé par précipitation puis filtration, le composé fluoré ramifié étant recueilli par des opérations de distillation.

Pour préparer un composé de la sous-famille (5), on fait réagir à chaud le sel de phosphonium sur une cétone de structure suivante :

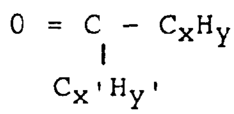

Les composés fluorés ramifiés conformes à l'invention peuvent être utilisés pour la préparation d'une composition artificielle d'humeur vitrée ou aqueuse pour un oeil, en particulier les composés (2) dans lesquels le groupement $C_xH_y$ est constitué par un ou des radicaux méthyle, éthyle et/ou propyle.

Les composés suivants :

(7)

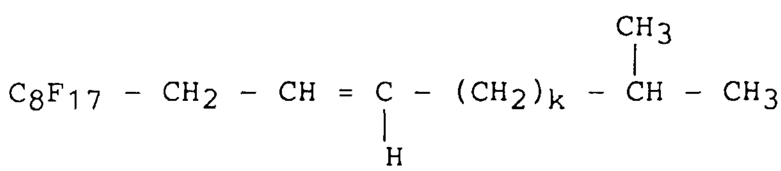

avec k = 1, 2 ou 3 sont particulièrement adaptés pour préparer une composition artificielle destinée à remplacer temporairement l'humeur vitrée d'un oeil en vue d'un recollement de rétine. La densité de ces composés est comprise entre 1,48 et 1,54 et l'indice de réfraction entre 1,33 et 1,35.

Dans les applications ophtalmologiques ou plus généralement dans les applications exigeant une pureté élevée, on complète le procédé de synthèse par les opérations finales de purification suivantes : extraction combinée en phases organique et aqueuse, adaptée pour éliminer les traces de solvant et d'oxyde de phosphine, élimination de la phase aqueuse par décantation, évaporation de la phase organique, distillation rapide sous vide avec refroidissement à l'azote du produit obtenu, et soumission du produit obtenu à des opérations de filtration et neutralisation pour éliminer les derniers résidus.

On a par ailleurs constaté que les composés fluorés ramifiés conformes à l'invention présentent une bonne

solubilité dans les huiles de silicone ou les huiles fluorosiliconées. Cette propriété permet, le cas écnéant, de les utiliser sous la forme d'une solution ou d'une suspension dans ce type d'huile, afin d'obtenir des produits ayant les propriétés des huiles de silicone ou des huiles fluorosiliconées et une densité ajustable supérieure ou égale à 1. Avantageusement, on utilise à cet effet des huiles présentant une viscosité comprise entre 100 cSt et 10 000 cSt. Par exemple, dans le domaine ophtalmologique, on peut utiliser les composés (2) ou (7) sus-évoqués en solution dans une huile de silicone ou une huile fluorosiliconée pour la préparation d'une composition artificielle, pour remplacer l'humeur vitrée ou aqueuse de l'oeil en vue de produire un effet de tamponnement interne de l'oeil. Ceci fournit un moyen supplémentaire pour abaisser la densité et l'ajuster à la valeur désirée, notamment pour des produits destinés à demeurer dans l'oeil pendant de longues durées. De plus, dans certaines applications ophtalmologiques, ceci permet aussi de combiner les propriétés des huiles de silicone avec les propriétés des composés de l'invention (viscosité, densité, indice de réfraction). Il convient de noter que la solubilité élevée des composés conformes à l'invention dans les huiles de silicone évite tout risque de formation d'une émulsion ou d'une interface dans la masse.

Les composés fluorés ramifiés conformes à l'invention peuvent également être utilisés dans toutes les applications où la capacité de solubiliser l'oxygène est recherchée, en particulier pour la préparation d'un milieu de conservation d'organes, tissus ou cellules biologiques, notamment milieu de conservation de tissus oculaires, tels que cornées.

Les exemples qui suivent sont destinés à illustrer l'invention.

EXEMPLE n° 1

FABRICATION DU COMPOSE

$$
\begin{array}{l}
\qquad\qquad\qquad\qquad\qquad\qquad\qquad\quad /CH_3 \\
C_8F_{17} - CH_2 - CH = CH - CH_2 - CH \\
\qquad\qquad\qquad\qquad\qquad\qquad\qquad\quad \backslash CH_3
\end{array}
$$

1ère étape : Fabrication du sel de phosphonium

Dans un réacteur de deux litres, on met 226 g de triphénylphosphine $(C_6H_5)_3P$ et 500 g d'iodure de fluoroalkyle $I - CH_2 - CH_2 - C_8F_{17}$.

On chauffe à 110° C pendant 12 heures sous agitation.

Le produit final prend en masse. On le lave au toluène puis à l'éther et on le sèche à 40° C sous vide ($10^3$Pa) pendant 12 heures.

Le rendement en sel de phosphonium de formule : $I^- (C_6H_5)_3 P^+ - CH_2 - CH_2 - C_8F_{17}$ est de l'ordre de 95 % (caractérisé par RMN et microanalyse).

2ème étape : réaction de Wittig

La réaction de Wittig est conduite dans des conditions de transfert de phase.

Dans un réacteur de deux litres, on met 512 g de sel de phosphonium obtenu à l'étape précédente, 42 g d'isovaléraldéhyde $(CH_3)_2 CH-CH_2-CHO$, 101 g de $K_2 CO_3$, 6,6 g d'eau et 488 ml de dioxane.

L'eau solvate le potassium K+ et libère l'ion $CO3^{2-}$ donnant des conditions basiques douces.

On chauffe à reflux du dioxane (100° C) pendant 18 heures sous agitation vigoureuse. On obtient un mélange pâteux brunâtre.

On élimine le $K_2 CO_3$ par filtration en le rinçant au dioxane.

On évapore le dioxane sous vide à froid ($10^3$ Pa) et on provoque la précipitation de l'oxyde de triphénylphosphine par ajout d'éther à froid (4° C).

On élimine le précipité par filtration et on évapore l'éther sous vide à froid ($10^3$ Pa).

Le produit (liquide ambre) subit ensuite une double extraction à la fois à l'eau et à l'hexane (1V/1V/1V). On élimine ainsi les dernières traces de dioxane et d'oxyde de triphénylphosphine.

La phase organique est lavée trois fois à l'eau (1V/1V).

On évapore l'hexane sous vide à froid ($10^3$ Pa). On fait ensuite une distillation rapide sous vide du type "bulbe à bulbe" : le piège est refroidi à l'azote liquide ; quand le vide atteint 1 à 10 Pa, on chauffe à 40° C, on élimine dans une première fraction les traces éventuelles de solvants et d'eau puis on récupère le composé organique fluoré hydrogéné synthétisé en chauffant à environ 100° C.

Le produit obtenu est transparent.

On le lave encore à l'eau et on le traite au charbon actif avant filtration sur un filtre à 0,22 μ.

Le rendement de la réaction en composé fluoré ramifié est d'environ 80 % ; le rendement global de la fabrication est donc de 76 %.

Il est à noter que ce rendement de fabrication est en valeur relative 50 % environ plus élevé que celui obtenu dans la synthèse du composé linéaire $C_8F_{17}$ - $CH_2$- CH = CH - $CH_2$- $CH_2$- $CH_2$- $CH_3$.

On obtient un liquide transparent que l'on peut conditionner en flacons.

<u>EXEMPLE n° 2</u>

CARACTERISATION DU COMPOSE

$$
\begin{array}{l} \qquad\qquad\qquad\qquad\qquad\qquad\quad /CH_3 \\ C_8F_{17} - CH_2 - CH = CH - CH_2 - CH \\ \qquad\qquad\qquad\qquad\qquad\qquad\quad \backslash CH_3 \end{array}
$$

Le composé organique fluoré hydrogéné obtenu par la synthèse décrite précédemment possède les caractéristiques physicochimiques suivantes (mesurées par densitométrie, réfractométrie, tensiométrie, viscosimétrie, ...).

Aspect liquide transparent

| | |
|---|---|
| Masse moléculaire | 516 |
| Indice de réfraction | 1,338 |
| Densité | 1,5 |
| Tension superficielle | 18 mN/m |
| (à 37° C mesurée à l'aide d'un tensiomètre automatique) | |
| Viscosité | < $5.10^{-6} m^2/s$ (soit 5 cSt) |
| Point d'ébullition | 110° C (à la pression de 1 Pa) |

Absorption d'$O_2$

- Spectre RMN de $C_8F_{17}$ - $CH_2$- CH = CH - $CH_2$- CH - $(CH_3)_2$
  RMN ($^{13}C$, 50 MHz, $CDCl_3$)

  ppm : 135,86 (s, 1C, $CH_\gamma C_8F_{17}$) ;
  122,65-111,20 (m, 8C, $C_8F_{17}$) ;
  116,1 (t, 1C, $CH_\beta C_8F_{17}$) ;
  36,36 (s, 1C, $CH_2$ en β double liaison) ;
  29,46 (t, 1C, $CH_2\alpha C_8F_{17}$) ;
  28,3 (s, 1C, $CH\alpha CH_3$) ;
  22,03 (s, 2C, $CH_3$) ;

  RMN ($^1H$, 80 MHz, $CD_3COCD_3$)

  ppm : 6-5,58 (sex, 1H, $CH_\beta C_8F_{17}$) ;
  5,56-5,31 (sex, 1H, $CH_\gamma C_8F_{17}$) ;
  3,31-2,75 (sex, 2H, $CH_2\alpha C_8F_{17}$) ;
  2,2-2 (sex, 2H, $CH_2$ en β double liaison) ;
  1,95-1,61 (m, 1H, $CH\alpha CH_3$) ;

0,96-0,88 (6H, m,$(CH_3)_2$)

- Spectre IR de $C_8F_{17}$ - $CH_2$- CH = CH - $CH_2$- CH - $(CH_3)_2$
  ϑ H-C = 3030 $cm^{-1}$ ; ϑ CH aliph 2870 $cm^{-1}$, 2930 $cm^{-1}$, 2960 $cm^{-1}$
  ϑ C = C 1660 $cm^{-1}$

EXEMPLE n° 3

APPLICATION DU COMPOSE

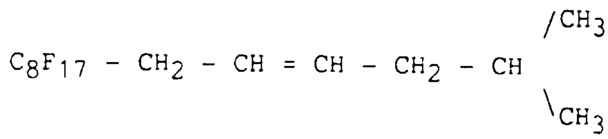

A LA CHIRURGIE DE LA RETINE.

Une étude expérimentale du composé a été faite comme substitut temporaire de l'humeur vitrée pour en vérifier la tolérance vis-à-vis de la rétine.

Chez le lapin, sous anesthésie générale, on effectue une vitrectomie la plus complète possible des globes oculaires et on injecte le composé à tester (à la place du vitré).

Deux temps de contact avec la rétine sont testés : 3 heures et 7 jours.

Pour le groupe 7 jours, un examen clinique ophtalmologique a lieu à J + 4, montrant un segment antérieur calme et un fond d'oeil normal dans tous les cas.

A l'issue de la période de test, les globes oculaires sont prélevés et fixés pour étude histologique.

On ne met en évidence aucune modification de la rétine ; il y a préservation des photorécepteurs et les couches nucléaires externes et internes sont bien organisées.

On conclut à une bonne tolérance intraoculaire du composé fluoré hydrogéné synthétisé.

EXEMPLE n° 4

PREPARATION D'UNE SOLUTION DE COMPOSE FLUORE RAMIFIE DANS UNE HUILE DE SILICONE.

On solubilise dans 130 g de polydiméthylsiloxane 23 g du composé précédemment préparé :

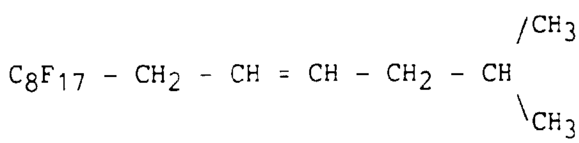

On agite et on chauffe légèrement pour favoriser la solubilisation.

On obtient une solution limpide visqueuse à 15 % en poids du composé fluoré, dont les caractéristiques sont les suivantes :

. aspect liquide transparent,
. indice de réfraction : 1,398,
. viscosité : $8,10^{-4} m^2/s$ (soit 800 cSt),
. densité : 1,04

EXEMPLE n° 5

SYNTHESE ET CARACTERISATION DU COMPOSE FLUORE HYDROGENE DE FORMULE :

$$C_8F_{17} - CH_2 - CH = CH - CH\begin{matrix}/CH_3\\ \backslash CH_3\end{matrix}$$

1ère étape : synthèse du sel de phosphonium fluoré

Dans un réacteur, on fait réagir 0,5 mole de triphénylphosphine $(C_6\ H_5)_3P$ et 0,5 mole d'iodure de fluoroalkyle $I\ CH_2\ CH_2\ C_8\ F_{17}$ à 95° C sans solvant.

Le produit est lavé au toluène puis à l'éther et séché sous vide ($10^3$ Pa) pendant 12 heures.

Le rendement en sel de phosphonium est de 95 %.

2ème étape :

Dans un réacteur, on fait réagir 0,025 mole de sel de phosphonium, 0,02 mole d'isobutyraldéhyde $(CH_3)_2$ - CH - CHO, 1,7 $10^{-2}$ moles de formamide, 0,03 mole de $K_2\ CO_3$ et 25 ml de dioxane sous agitation à reflux du dioxane pendant 5 heures.

Après élimination du $K_2\ CO_3$, précipitation de l'oxyde triphénylphosphine, extraction, filtration et distillation "rapide", on obtient avec un rendement de 60 % un produit final incolore qui a les caractéristiques suivantes :

- . Aspect liquide transparent
- . Poids moléculaire 502
- . Densité 1,5
- . Indice de réfraction 1,34
- . Viscosité $< 5.10^{-6} m^2/s$ (soit 5 cSt)
- . Tension superficielle 18 mN/m (à 37° C)

- Spectre RMN
  RMN ($^{13}C$, 50 MHz, $CDCl_3$)

  ppm : 144,20 (s, 1C, $CH_\gamma C_8F_{17}$) ;
  122,65-111,20 (m, 8C, $C_8F_{17}$) ;
  112,75 (t, 1C, $CH_\beta C_8F_{17}$) ;
  29,35 (t, 1C, $CH_2\alpha C_8F_{17}$) ;
  26,71 (s, 1C, $CH_\alpha CH_3$) ;
  22,07 (s, 2C, $(CH_3)_2$) ;

  RMN ($^1H$, 80 MHz, $CD_3COCD_3$)

  ppm : 5,95-5,72 (sex, 1H, $CH_\beta C_8F_{17}$) ;
  5,55-5,29 (sex, 1H, $CH_\gamma C_8F_{17}$) ;
  3,5-2,9 (sex, 2H, $CH_2\alpha C_8F_{17}$) ;
  3-2,5 (m, 1H, $CH\alpha CH_3$) ;
  1,16-1,08 (d, 6H, $CH_3$

**Revendications**

1. Composé organique fluoré, possédant la structure moléculaire suivante :

(1)

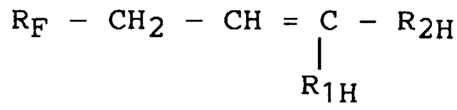

où $R_F$ est une chaîne carbonée fluorée, $R_{1H}$ est un hydrogène ou une chaîne carbonée hydrogénée saturée, $R_{2H}$ est un hydrogène ou une chaîne carbonée hydrogénée saturée, $R_{1H}$ et $R_{2H}$ ne sont pas simultanément des hydrogènes, le groupement

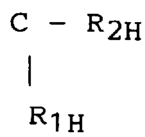

comporte une chaîne principale et au moins une ramification linéaire ou cyclique.

**2.** Composé fluoré ramifié selon la revendication 1, dans lequel $R_{1H}$ est un hydrogène, possédant la structure moléculaire suivante :

(2)

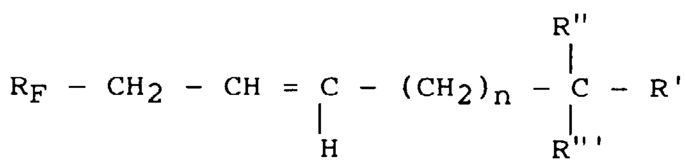

où les groupements R', R" et R"', identiques ou non, ont chacun la forme suivante : $(CH_2)_m$ - $C_xH_y$ avec :

$$n \geq 0$$

$$m \geq 0$$

$$y = 2x + 1 \text{ avec } x \geq 0$$

au moins deux des groupements R', R" et R"' n'étant pas des hydrogènes,
le nombre total de carbones des groupements R', R" et R"' étant inférieur à 25-n.

**3.** Composé fluoré ramifié selon la revendication 2, dans lequel le groupement $C_xH_Y$ est constitué par un ou des radicaux méthyle, éthyle et/ou propyle.

**4.** Composé fluoré ramifié selon la revendication 3, possédant la structure moléculaire suivante :

(3)

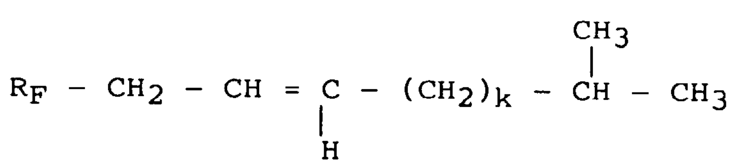

où k = 1, 2 ou 3.

**5.** Composé fluoré ramifié selon la revendication 2, possédant la structure moléculaire suivante :

(4) 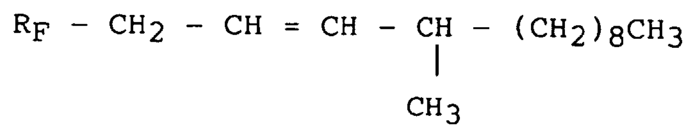

**6.** Composé fluoré ramifié selon la revendication 1, dans lequel $R_{1H}$ et $R_{2H}$ ne sont pas des hydrogènes, possédant la structure moléculaire suivante :

(5) 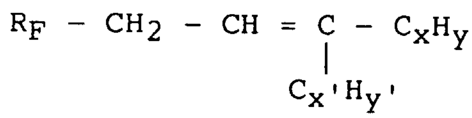

où

$$y = 2x+1 \text{ avec } x > 1$$

$$y' = 2x' \text{ ou } 2x'+1 \text{ avec } x' \geq 1$$

**7.** Composé fluoré ramifié selon la revendication 6, dans lequel les groupements $C_xH_y$ et $C_{x'}H_{y'}$ sont des radicaux méthyle, éthyle et/ou propyle.

**8.** Composé fluoré ramifié selon l'une des revendications 1 à 7, dans lequel la chaîne fluorée $R_F$ est une chaîne perfluorée possédant la formule $C_pF_{2p+1}$ avec $2 \leq p \leq 12$.

**9.** Composé fluoré ramifié selon la revendication 8, possédant une densité comprise entre 1,1 et 2,2 fonction croissante de p.

**10.** Composé fluoré ramifié selon la revendication 8, possédant un indice de réfraction compris entre 1,2 et 1,7 fonction de p et du nombre de carbones de $R_{1H}$ et $R_{2H}$.

**11.** Composé fluoré ramifié conforme à l'une des revendications 3 ou 4, dans lequel la chaîne fluorée $R_F$ est une chaîne perfluorée possédant la formule $C_pF_{2p+1}$ avec $2 \leq p \leq 12$, possédant une solubilité dans l'huile de silicone supérieure à 10 % en poids, fonction croissante de k et fonction décroissante de p.

**12.** Composé fluoré ramifié selon l'une des revendications 8 à 11, dans lequel la chaîne perfluorée $R_F$ est $C_8F_{17}$.

**13.** Composé fluoré ramifié selon la revendication 12, de formule :

(6) 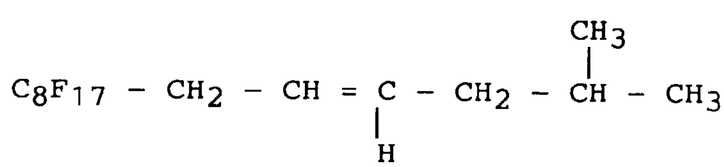

possédant une densité sensiblement égale à 1,5 et un indice de réfraction sensiblement égal à 1,338.

**14.** Utilisation d'un composé fluoré ramifié conforme à l'une des revendications 3 ou 4 pour la préparation d'une composition artificielle d'humeur vitrée ou aqueuse pour un oeil.

**15.** Utilisation du composé fluoré ramifié :

(7)

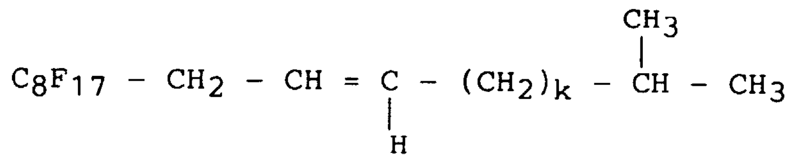

avec k = 1, 2 ou 3, pour la préparation d'une composition artificielle destinée à remplacer temporairement l'humeur vitrée ou aqueuse d'un oeil en vue d'un recollement de rétine.

**16.** Solution ou suspension du composé fluoré ramifié conforme à l'une des revendications 1 à 13 dans une huile de silicone ou une huile fluorosiliconée.

**17.** Utilisation d'une solution ou d'une suspension conforme à la revendication 16 pour la préparation d'une composition artificielle, pour remplacer l'humeur vitrée ou aqueuse de l'oeil en vue de produire un effet de tamponnement interne de l'oeil.

**18.** Utilisation d'un composé fluoré ramifié conforme à l'une des revendications 1 à 13 pour la préparation d'un milieu de conservation d'organes, tissus ou cellules biologiques, en particulier de tissus oculaires tels que cornées.

**19.** Procédé de préparation d'un composé fluoré ramifié conforme à la revendication 1, caractérisé par les opérations suivantes :

- on fait réagir à chaud une phosphine $Z_3P$ sur un halogénure de formule $XCH_2CH_2R_F$, de façon à obtenir un sel de phosphonium $Z_3P^+X^-CH_2CH_2R_F$,
- et on fait réagir à chaud dans un solvant ledit sel de phosphonium sur un aldéhyde ramifié ou une cétone

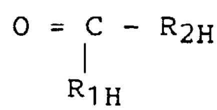

en présence d'une base faible.

**20.** Procédé selon la revendication 19 pour préparer un composé fluoré ramifié possédant la structure moléculaire suivante :

(2)

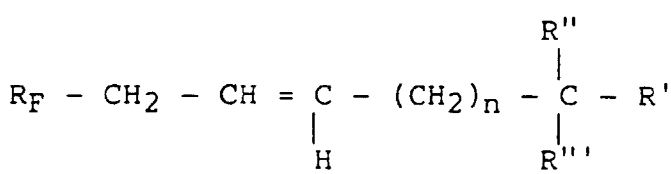

où les groupements R', R" et R"', identiques ou non, ont chacun la forme suivante : $(CH_2)_m - C_xH_y$ avec :

$$n \geq 0$$

$$m \geq 0$$

$$y = 2x + 1 \text{ avec } x > 0$$

au moins deux des groupements R', R" et R"' n'étant pas des hydrogènes,
le nombre total de carbones des groupements R', R" et R"' étant inférieur à 25-n,

dans lequel on fait réagir à chaud le sel de phosphonium sur un aldéhyde ramifié de structure suivante :

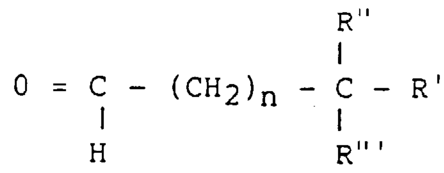

**21.** Procédé de préparation selon la revendication 20, dans lequel :

- . on fait réagir la triphénylphosphine $(C_6H_5)_3$ P sur un iodure de fluoroalkyle,
- . on fait réagir le sel de phosphonium obtenu avec l'aldéhyde ramifié suivant :

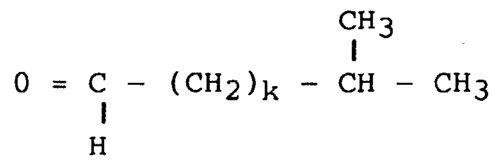

avec k = 1, 2 ou 3, le solvant utilisé étant du dioxane en présence de carbonate de potassium et d'eau jouant le rôle de catalyseur, la température étant ajustée à la température de reflux du dioxane,

- . on élimine le carbonate de potassium par filtration,
- . on provoque la précipitation de l'oxyde de triphénylphosphine formé,
- . après filtration, on recueille le composé fluoré ramifié par des opérations de distillation.

**22.** Procédé selon la revendication 19 pour préparer un composé fluoré ramifié possédant la structure moléculaire suivante :

(5)

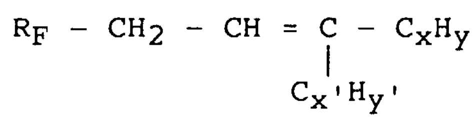

où

$$y = 2x+1 \text{ avec } x \geq 1$$

$$y' = 2x'+1 \text{ avec } x' \geq 1$$

dans lequel on fait réagir à chaud le sel de phosphonium sur une cétone de structure suivante :

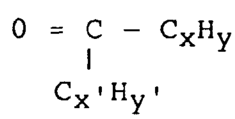

**23.** Procédé selon la revendication 21, permettant d'obtenir un composé fluoré purifié liquide incolore, dans lequel on réalise, en étapes finales, une extraction combinée en phases organique et aqueuse, adaptée pour éliminer les traces de dioxane et d'oxyde de triphénylphosphine, on élimine la phase aqueuse par décantation, on évapore la phase organique, on réalise une distillation rapide sous vide avec refroidissement à l'azote du produit obtenu, et

on fait subir au produit obtenu des opérations de filtration et neutralisation pour éliminer les derniers résidus.

**Patentansprüche**

1. Organische Fluorverbindung mit der folgenden Molekularstruktur:

(1) 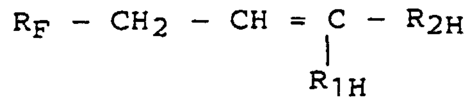

wobei $R_F$ eine Fluorkohlenstoffkette, $R_{1H}$ Wasserstoff oder eine gesättigte Kohlenwasserstoffkette, $R_{2H}$ Wasserstoff oder eine gesättigte Kohlenwasserstoffkette ist, $R_{1H}$ und $R_{2H}$ nicht gleichzeitig Wasserstoff sind, die Gruppe

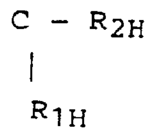

eine Hauptkette und wenigstens eine lineare oder zyklische Verzweigung enthält.

2. Verzweigte Fluorverbindung nach Anspruch 1, bei der $R_{1H}$ Wasserstoff ist, mit der folgenden Molekularstruktur:

(2) 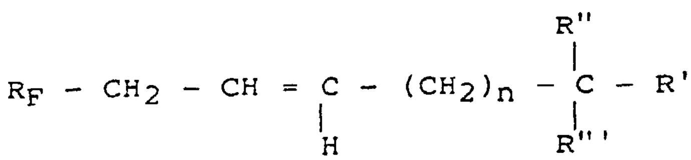

wobei die Gruppen R', R" und R"', identisch oder nicht, jeweils die folgende Formel haben: $(CH_2)_m$ - $C_xH_y$, wobei:

$$n \geq 0$$

$$m \geq 0$$

$$y = 2x + 1, \text{ wobei } x \geq 0 \text{ ist,}$$

wobei wenigstens zwei der Gruppen R', R" und R"' nicht Wasserstoff sind,
wobei die Gesamtzahl der Kohlenstoffatome der Gruppen R', R" und R"' kleiner als 25-n ist.

3. Verzweigte Fluorverbindung nach Anspruch 2, bei der die Gruppe $C_xH_y$ aus einem oder mehreren Methyl-, Ethyl- und/oder Propylradikalen besteht.

4. Verzweigte Fluorverbindung nach Anspruch 3, mit der folgenden Molekularstruktur:

(3)

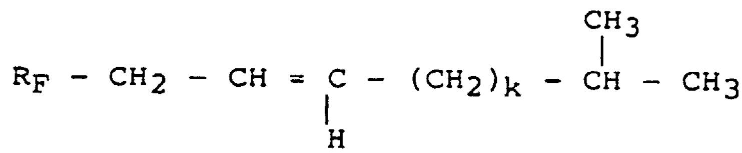

wobei k = 1, 2 oder 3.

**5.** Verzweigte Fluorverbindung nach Anspruch 2, mit der folgenden Molekularstruktur:

(4)

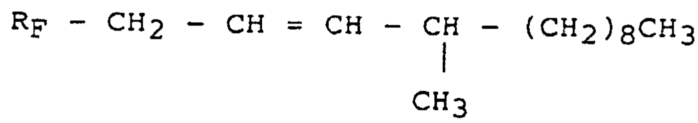

**6.** Verzweigte Fluorverbindung nach Anspruch 1, bei der $R_{1H}$ und $R_{2H}$ nicht Wasserstoff sind, mit der folgenden Molekularstruktur:

(5)

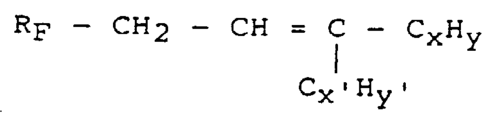

wobei

$$y = 2x+1, \text{ wobei } x \geq 1$$

$$y' = 2x' \text{ oder } 2x'+1, \text{ wobei } x' \geq 1 \text{ ist.}$$

**7.** Verzweigte Fluorverbindung nach Anspruch 6, bei der die Gruppen $C_xH_y$ und $C_x'H_y'$ Methyl-, Ethyl- und/oder Propylradikale sind.

**8.** Verzweigte Fluorverbindung nach einem der Ansprüche 1 bis 7, bei der die Fluorkette $R_F$ eine Perfluorkette der Formel $C_pF_{2p+1}$ ist, wobei $2 \leq p \leq 12$.

**9.** Verzweigte Fluorverbindung nach Anspruch 8, die eine Dichtigkeit zwischen 1,1 und 2,2 besitzt, mit ansteigender Abhängigkeit von p.

**10.** Verzweigte Fluorverbindung nach Anspruch 8, mit einem Beugungsindex zwischen 1,2 und 1,7, mit Abhängigkeit von p und einer Kohlenstoffatomzahl von $R_{1H}$ und $R_{2H}$.

**11.** Verzweigte Fluorverbindung nach einem der Ansprüche 3 und 4, bei der die Fluorkette $R_F$ eine Perfluorkette der Formel $C_pF_{2p+1}$ ist, wobei $2 \leq p \leq 12$, mit einer Silikonöllöslichkeit von mehr als 10 Gew.-%, mit ansteigender Abhängigkeit von k und abnehmender Abhängigkeit von p.

**12.** Verzweigte Fluorverbindung nach einem der Ansprüche 8 bis 11, bei der die Perfluorkette $R_F$ $C_8F_{17}$ ist.

**13.** Verzweigte Fluorverbindung nach Anspruch 12 mit der Formel:

(6) 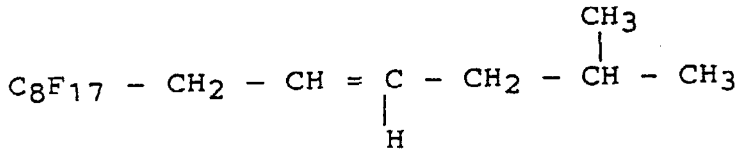

mit einer Dichte im wesentlichen von 1,5 und einem Beugungsindex von im wesentlichen 1,338.

**14.** Verwendung einer verzweigten Fluorverbindung nach einem der Ansprüche 3 und 4 für die Herstellung einer künstlichen Struktur eines Glas- oder Wasserkörpers für ein Auge.

**15.** Verwendung der verzweigten Fluorverbindung:

(7) 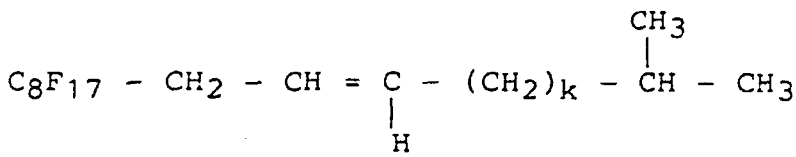

wobei k = 1, 2 oder 3, für die Herstellung einer künstlichen Struktur zum vorübergehenden Ersetzen des Glas- oder Wasserkörpers eines Auges mit dem Ziel, die Retina wiederherzustellen.

**16.** Lösung oder Suspension der verzweigten Fluorverbindung nach einem der Ansprüche 1 bis 13 in einem Silikonöl oder einem Fluorsilikonöl.

**17.** Verwendung einer Lösung oder einer Suspension nach Anspruch 16 für die Herstellung einer künstlichen Struktur zum Ersetzen des Glas- oder Wasserkörpers des Auges mit dem Ziel, einen Zusammenstoßeffekt im Innern des Auges zu erzeugen.

**18.** Verwendung einer verzweigten Fluorverbindung nach einem der Ansprüche 1 bis 13 für die Herstellung einer Konservierungsumgebung für Organe, Gewebe oder biologische Zellen, insbesondere von Augengeweben wie Hornhäuten.

**19.** Verfahren zur Herstellung einer verzweigten Fluorverbindung nach Anspruch 1, gekennzeichnet durch die folgenden Vorgänge:

Reagieren eines Phosphins $Z_3P$ unter Wärme auf einem Halogenid der Formel $XCH_2CH_2R_F$, um ein Salz des Phosphoniumsalz $Z_3P^+X^-CH_2R_F$ zu erhalten,
und Reagieren des genannten Phosphoniumsalzes unter Wärme in einem Lösungsmittel auf einem verzweigten Aldehyd oder einem Keton

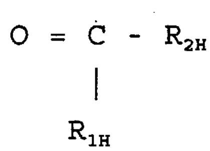

in Anwesenheit einer schwachen Base.

**20.** Verfahren nach Anspruch 19 zur Herstellung einer verzweigten Fluorverbindung der folgenden Molekularstruktur:

(2)

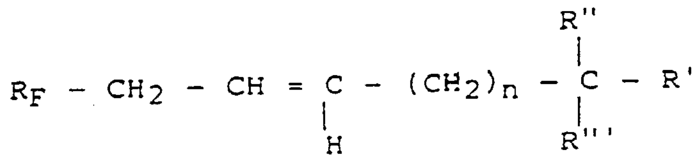

wobei die Gruppen R', R" und R"', identisch oder nicht, jeweils die Formel $(CH_2)_m - C_xH_y$ haben, wobei:

$$n \geq 0$$

$$m \geq 0$$

$$y = 2x + 1, \text{ wobei } x \geq 0$$

wobei wenigstens zwei der Gruppen R', R" und R"' nicht Wasserstoff sind,
wobei die Gesamtzahl der Kohlenstoffatome der Gruppen R', R" und R"' kleiner ist als 25-n,
wobei das Phosphoniumsalz auf einem verzweigten Aldehyd der folgenden Struktur unter Wärme reagieren gelassen wird:

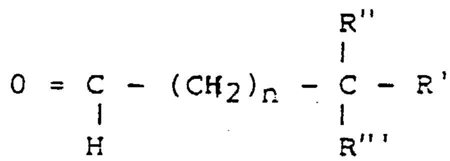

**21.** Verfahren zur Herstellung nach Anspruch 20, bei dem das Triphenylphosphin $(C_6H_5)_3P$ auf einem Fluoralkyljod reagieren gelassen wird,

das erhaltene Phosphoniumsalz mit dem folgenden verzweigten Aldehyd reagieren gelassen wird:

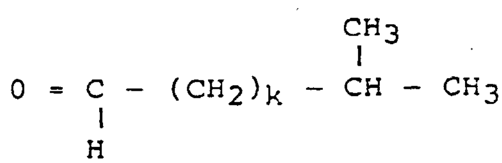

wobei k = 1, 2 oder 3, wobei das verwendete Lösungsmittel Dioxan in Anwesenheit von Kaliumkarbonat ist, und wobei Wasser als Katalysator eingesetzt wird, wobei die Temperatur auf die Rückflußtemperatur von Dioxan eingestellt wird,
Eliminieren des Kaliumkarbonats durch Filtration,
Bewirken einer Ausfällung des gebildeten Triphenylphosphinoxids,
nach der Filtration Auffangen der verzweigten Fluorverbindung durch Destillationsvorgänge.

**22.** Verfahren nach Anspruch 19 für die Herstellung einer verzweigten Fluorverbindung mit der folgenden Molekularstruktur:

(5) 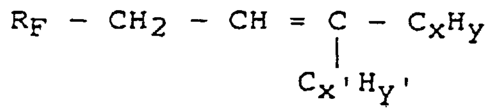

wobei

$$y = 2x+1, \text{ wobei } x \geq 1$$

$$y' = 2x'+1, \text{ wobei } x' \geq 1$$

wobei das Phosphoniumsalz auf einem Keton der folgenden Struktur reagieren gelassen wird:

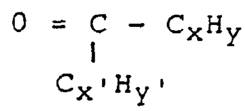

**23.** Verfahren nach Anspruch 21, das den Erhalt einer farblosen gereinigten flüssigen Fluorverbindung zuläßt, wobei in der letzten Phase die folgenden Schritte durchgeführt werden: eine kombinierte Extraktion der organischen und der wäßrigen Phase, um die Dioxan- und Triphenylphosphinoxidspuren zu eliminieren, Eliminieren der wäßrigen Phase durch Dekantierung, Verdunstenlassen der organischen Phase, Durchführen einer schnellen Destillation unter Vakuum mit Kühlung des erhaltenen Produkts unter Stickstoff, und Unterziehen des Produkts einer Filtration oder Neutralisation, um die letzten Rückstände zu eliminieren.

**Claims**

**1.** The fluorinated organic compound having the following molecular structure:

(1) 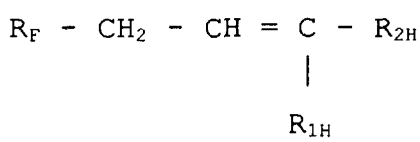

where $R_F$ is a fluorinated carbon chain, $R_{1H}$ is hydrogen or a saturated hydrogenated carbon chain, $R_{2H}$ is hydrogen or a saturated hydrogenated carbon chain, $R_{1H}$ and $R_{2H}$ being not simultaneously hydrogen, the group

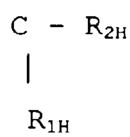

comprising a principal chain and at least one linear or cyclic branch.

**2.** The branched fluorinated compound according to claim 1, wherein $R_{1H}$ is hydrogen, with the following molecular structure:

(2) 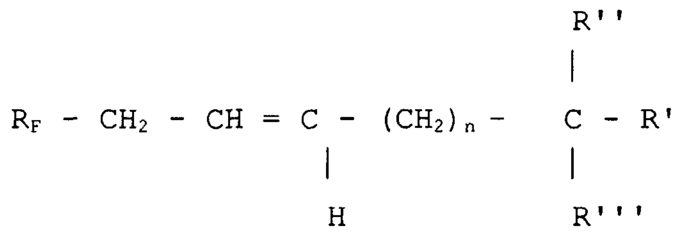

where the groups R', R" and R"', identical or not, have each the following formula: $(CH_2)_m$ - $C_xH_y$, with:

$$n \geq 0$$

$$m \geq 0$$

$$y = 2x+1 \text{ with } x \geq 0$$

at least two of the groups R', R" and R"' not being hydrogen atoms,
the total number of carbon atoms of the groups R', R" and R"' being lower than 25-n.

**3.** The branched fluorinated compound according to claim 2, wherein the $C_xH_y$ group consists of one or more methyl, ethyl or propyl groups.

**4.** The branched fluorinated compound according to claim 3, having the following molecular structure:

(3) 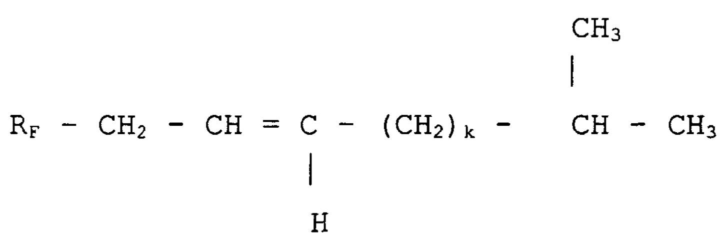

where k = 1, 2, or 3.

**5.** The branched fluorinated compound according to claim 2, having the following molecular structure:

(4) 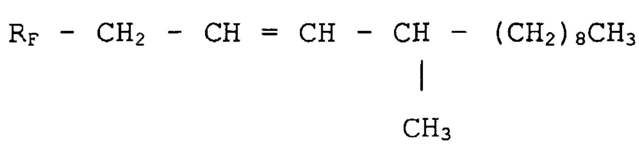

**6.** The branched fluorinated compound according to claim 1, wherein $R_{1H}$ and $R_{2H}$ are both other than hydrogen, the compound having the following molecular structure:

(5) $R_F - CH_2 - CH = C - C_xH_y$

$|$

$C_{x'}H_{y'}$

where

$$y = 2x+1, \text{ with } x \geq 1,$$

$$y' = 2x' \text{ or } 2x'+1, \text{ with } x' \geq 1.$$

7. The branched fluorinated compound according to claim 6, wherein the groups $C_xH_y$ and $C_{x'}H_{y'}$ are methyl, ethyl or propyl groups.

8. The branched fluorinated compound according to one of the claims 1 to 7, wherein the fluorinated chain $R_F$ is a perfluorinated chain having the formula $C_pF_{2p+1}$ with $2 \leq p \leq 12$.

9. The branched fluorinated compound according to claim 8, having a density in the range between 1.1 and 2.2, said density being an increasing function of p.

10. The branched fluorinated compound according to claim 8, having a refractive index in the range between 1.2 and 1.7, said refractive index being an increasing function of p and the number of carbon atoms in $R_{1H}$ and $R_{2H}$.

11. The branched fluorinated compound according to one of the claims 3 or 4, wherein the fluorinated chain $R_F$ is a perfluorinated chain having the formula $C_pF_{2p+1}$ with $2 \leq p \leq 12$, with a solubility in silicone oil greater than 10 wt %, said solubility being an increasing function of k and a decreasing function of p.

12. The branched fluorinated compound according to one of the claims 8 to 11, wherein the fluorinated chain $R_F$ is $C_8F_{17}$.

13. The branched fluorinated compound according to claim 12, having the formula:

$CH_3$

$|$

(6) $C_8F_{17} - CH_2 - CH = C - CH_2 - CH - CH_3$

$|$

H

and having a density of essentially 1.5 and a refractive index of essentially 1.338.

14. The use of a branched fluorinated compound according to one of the claims 3 or 4, for preparing an artificial composition of vitrous or aqeous humor for an eye.

15. The use of the branched fluorinated compound:

(7) 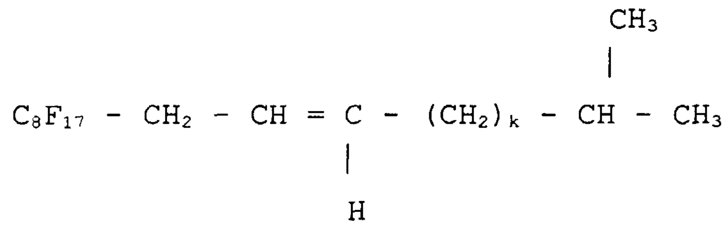

where k = 1, 2, or 3, for preparing an artificial composition for the temporary replacement of the vitrous or aqueous humor in the eye in view of a re-adhesion of the retina.

**16.** A solution or suspension of the branched fluorinated compound according to one of the claims 1 to 13 in a silicone or fluorosilicone oil.

**17.** The use of a solution or suspension according to claim 16, for preparing an artificial composition intended to replace the vitrous or aqueous humor in the eye, in order to provide an internal buffer effect in the eye.

**18.** The use of the branched fluorinated compound according to one of the claims 1 to 13 for preparing a medium for the preservation of biological organs, tissues or cells, particularly of ocular tissues such as corneae.

**19.** A method for preparing a branched fluorinated compound according to claim 1, characterized in that it comprises the steps of:

- reacting a phosphine $Z_3P$ under heating with a halogenide of formula $XCH_2CH_2R_F$ in order to obtain a phosphonium salt $Z_3P^+X^-CH_2CH_2R_F$,
- and reacting said phosphonium salt under heating in a solvent with a branched aldehyde or ketone

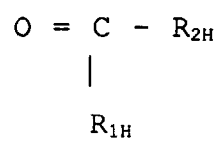

in the presence of a weak base.

**20.** The method according to claim 19, for preparing a branched fluorinated compound having the following molecular structure:

(2) 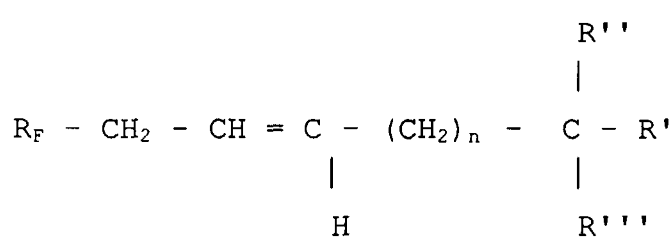

where the groups R', R" and R"', identical or not, have each the following formula: $(CH_2)_m - C_xH_y$, with:

$$n \geq 0$$

$$m \geq 0$$

$$y = 2x+1 \text{ with } x \geq 0$$

at least two of the groups R', R" and R'" not being hydrogen atoms,
the total number of carbon atoms of the groups R', R" and R'" being lower than 25-n,
wherein said phosphonium salt is reacted under heating with a branched aldehyde of the following formula:

(2) 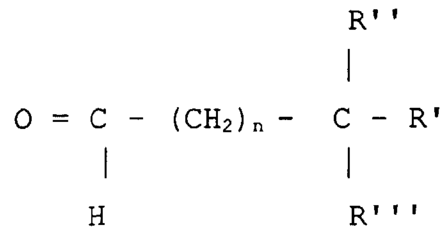

**21.** The preparation method according to claim 20, wherein:

- the triphenylphosphine $(C_6H_5)_3P$ is reacted with a fluoroalkyl iodide,
- the resultant phosphonium salt is reacted with the following branched aldehyde:

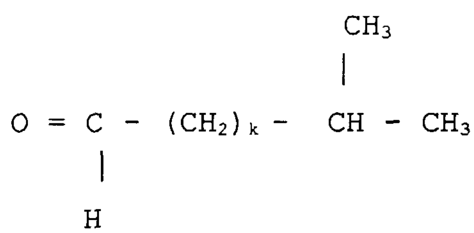

where k = 1, 2, or 3, dioxane being preferably used as a solvent, in the presence of potassium carbonate and water acting as a catalyst, the reaction temperature being adjusted to the reflux temperature of the dioxane,
- the potassium carbonate is removed by filtration,
- the resultant triphenylphosphine oxide is caused to precipitate,
- after filtration, the branched fluorinated compound is collected by means of distillation steps.

**22.** The method according to claim 19, for preparing a branched fluorinated compound having the following molecular structure:

(5) 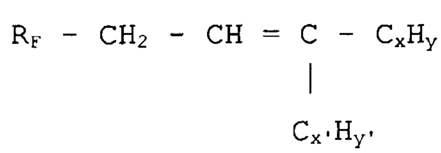

where

$$y = 2x+1, \text{ with } x \geq 1,$$

$$y' = 2x'+1, \text{ with } x' \geq 1,$$

wherein said phosphonium salt is reacted under heating with a ketone of the following formula:

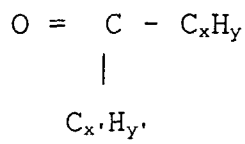

**23.** The method according to claim 21, for obtaining a colorless liquid purified fluorinated compound, wherein during final steps, a combined extraction in organic and aqueous phases, adapted for eliminating the remaining traces of the dioxane and triphenylphosphine oxide is conducted, the aqueous phase is removed by means of decantation, the organic phase is evaporated, a flash distillation under vacuum and cooling of the resultant product with nitrogen are carried out, and the resultant product is subjected to filtration and neutralization steps in order to remove the last residues.